# EUROPÄISCHE PATENTSCHRIFT

(11) **EP 2 747 756 B1**
(45) Veröffentlichungstag und Bekanntmachung des Hinweises auf die Patenterteilung: **29.07.2015**
(21) Anmeldenummer: 12798259.3
(22) Anmeldetag: 30.11.2012
(51) Int. Cl.: A61K 31/00, A61K 31/191, A61K 31/194, A61K 31/198

(54) **METABOLISIERBARE SALZE UND DEREN VERWENDUNG IN DIAGNOSTIK UND THERAPIE**
METABOLIZABLE SALTS AND USE THEREOF IN DIAGNOSTICS AND THERAPY
SELS MÉTABOLISABLES ET LEUR UTILISATION POUR LE DIAGNOSTIC ET LA THÉRAPIE

(30) Priorität: 07.12.2011 DE 102011056142
(43) Veröffentlichungstag der Anmeldung: 02.07.2014
(73) Patentinhaber: Rüdinger, Manfred, 79224 Umkirch (DE)
(72) Erfinder: ZANDER, Rolf, 55122 Mainz (DE)
(74) Vertreter: WSL Patentanwälte Partnerschaft mbB
(86) Internationale Anmeldenummer: PCT/EP2012/074103
(87) Internationale Veröffentlichungsnummer: WO 2013/083489

(56) Entgegenhaltungen:
- EP-A2- 0 313 808
- WO-A1-00/78270
- WO-A2-2006/012615
- WO-A2-2006/044908
- DE-T2- 69 321 007
- T LE BRICON ET AL: "Ornithine [alpha] -ketoglutarate metabolism after enteral administration in burn patients: Bolus compared with continuous infusion", AMERICAN JOURNAL OF CLINICAL NUTRITION, Bd. 65, Nr. 2, 1. Januar 1997 (1997-01-01) , Seiten 512-518, XP055051364, ISSN: 0002-9165
- DE BANDT JEAN-PASCAL ET AL: "A randomized controlled trial of the influence of the mode of enteral ornithine alpha-ketoglutarate administration in burn patients", JOURNAL OF NUTRITION, Bd. 128, Nr. 3, März 1998 (1998-03), Seiten 563-569, XP002691062, ISSN: 0022-3166
- DATABASE MEDLINE [Online] US NATIONAL LIBRARY OF MEDICINE (NLM), BETHESDA, MD, US; August 2005 (2005-08), PENG XI ET AL: "[Comparative study on the influence of arginine hydrochloride and arginine acetate on the immune function and acid-base balance in rabbits with severe burns].", XP002691063, Database accession no. NLM16185408
- SZÁM I ET AL: "Therapie experimenteller und klinischer Hyperannonämien mit Ornithin-alpha-Ketoglutarat = Therapy of experimental and clinical hyperammonaemia with ornithine-alpha-ketoglutarate", WIENER MEDIZINISCHE WOCHENSCHRIFT (1946), SPRINGER WIEN, AT, Bd. 124, Nr. 20, 18. Mai 1974 (1974-05-18) , Seiten 319-325, XP009144470, ISSN: 0043-5341
- GÁBOR ORGOVÁN ET AL: "The complete microspeciation of arginine and citrulline", JOURNAL OF PHARMACEUTICAL AND BIOMEDICAL ANALYSIS, Bd. 54, Nr. 5, 1. April 2011 (2011-04-01), Seiten 965-971, XP055051374, ISSN: 0731-7085, DOI: 10.1016/j.jpba.2010.11.023

## Beschreibung

Die vorliegende Erfindung betrifft Salzverbindungen zur Verwendung bei der Diagnose oder Therapie eines Patienten sowie Arzneimittel, Medizinprodukte, diagnostische Zusammensetzungen oder Blutprodukte, die eine Salzverbindung gemäß der vorliegenden Erfindung enthalten.

Bei jeglicher Zufuhr von größeren Flüssigkeitsmengen in den Körper eines Patienten ist darauf zu achten, dass eine möglichst balancierte Elektrolytlösung verwendet wird, die das physiologische Elektrolytmuster des Plasmas mit Natrium, Kalium, Calcium und Magnesium sowie Chlorid und ihren Beiträgen zur Osmolalität sowie einen physiologischen Säure-Basen-Status mit Bicarbonat oder ersatzweise anderen geeigneten Anionen aufweist. Die Infusion einer auf diese Weise balancierten Lösung kann daher - außer beim Volumen - keinen therapeutischen Fehler verursachen. Es ist daher eine Aufgabe der vorliegenden Erfindung für die Erzeugung von möglichst balancierten Elektrolytlösungen besonders gut geeignete Salzverbindungen bereitzustellen.

Andererseits gibt es selbstverständlich auch Formen der Flüssigkeitstherapie, bei der anders als mit physiologisch zusammengesetzten Lösungen gearbeitet wird, um einen vom Idealzustand abweichenden Zustand zu korrigieren oder vorbeugend entgegen eines in eine andere Richtung arbeitenden Mittels oder Umstandes die physiologischen Verhältnisse aufrecht zu erhalten. Entscheidend in den genannten Fällen ist, dass bei jeder Gabe von Elektrolyten enthaltenden Lösungen oder von Lösungen, die den Elektrolythaushalt beeinflussen können, dies in einer Weise geschieht, bei der insgesamt nicht nur das gewünschte Elektrolytmuster sondern auch das gewünschte Säure-Basen-Gleichgewicht und die ideale Osmolalität erreicht wird, und es ist daher eine Aufgabe der vorliegenden Erfindung auch hierfür geeignete Salzverbindungen bereitzustellen.

Theoretisch wäre Ammonium-Bicarbonat (auch Ammoniumhydrogencarbonat oder NH₄HCO₃) zur Elimination der beiden gasförmigen Stoffwechselendprodukte CO₂ und NH₃ und zusätzlich als physiologische Puffersubstanz im Körperflüssigkeitshaushalt geeignet. Ammonium-Bicarbonat weist mit seinem pH-Wert von 7,4 - 7,6 (je nach Ionenstärke) ideale Eigenschaften als physiologische Puffersubstanz auf. Der pH-Wert von Ammonium-Bicarbonat resultiert aus den pK-Werten der beiden hydratisierten Untereinheiten und den daraus entstehenden Anionen HCO₃⁻ (pK 6,1) und NH₄⁺ (pK 9,0).

Im neutralen pH-Bereich von 6 bis 8 und insbesondere im physiologischen pH-Bereich von 7,4 ± 0,4 gibt es kaum Puffersubstanzen, die therapeutisch einsetzbar wären. Synthetische, insbesondere in der Laborbiologie eingesetzte Puffer in dem relevanten pH-Bereich sind z.B. Hepes mit einem fast idealen pK von 7,35 sowie Tris mit einem pK von 8,2. Hepes wird aber nicht beim Menschen eingesetzt. Als Medikamente zugelassen kommen am Menschen nur Tris - zur Therapie einer Azidose - und Arginin-Hydrochlorid - zur Therapie einer Alkalose - zum Einsatz.

Eine Aufgabe der vorliegenden Erfindung besteht daher darin, für die Therapie des Flüssigkeitshaushaltes eines Patienten geeignete Salzverbindungen bereitzustellen, bei deren Verwendung der Elektrolythaushalt, das Säure-Basen-Gleichgewicht und/oder die Osmolalität in dem zu behandelnden Patienten in der gewünschten Weise eingestellt werden.

Neben der gezielten Beeinflussung des Flüssigkeitshaushaltes, wie z.B. bei der Infusionstherapie, gibt es eine Reihe von therapeutischen Eingriffen, die den Flüssigkeitshaushalt ungewollt beeinflussen. Beispielsweise werden bei der maschinellen Herstellung von Blutplasma oder Blutzellkonzentraten (Plasma- bzw. Cytapherese) dem extrakorporal durch das Gerät geführten Blutstrom zur Verringerung der Blutgerinnungsneigung häufig größere Mengen an Citrat zugeführt. Das Citrat wird üblicherweise als Natriumcitrat plus Citronensäure zugeführt, z.B. in der Form von ACD-A.

ACD-A (Acid-Citrate-Dextrose Typ A) ist eine Lösung von Citronensäure, Natriumcitrat und D-Glucose in Wasser. ACD-A verhindert die Gerinnung des Blutes, in dem die für die Blutgerinnung essentiellen Ca²⁺-Ionen komplexiert (gebunden) werden. ACD-A wird traditionell bei der Herstellung und Lagerung von Blutprodukten zur Gerinnungshemmung eingesetzt.

ACD-A ist sowohl *in vitro* (im Labor) als auch *in vivo* (im Patienten) eine stark hypotone Lösung. ACD-A erzeugt bei der Diagnostik *in vitro* im Blut eine Azidose mit einem BE (base excess; s.u.) von -13,8 mmol/l nach Verdünnung 1:10 (9 Teile Blut + 1 Teil Lösung). Bei der Verwendung zur Blutgerinnungshemmung im Zusammenhang mit der extrakorporalen Blutbehandlung kann ACD-A einen stark positiven BEpot (potentieller base excess; s.u.) beim Patienten erzeugen, je nachdem wie viel von den Bestandteilen der ACD-A-Lösung beim Patienten ankommen. Darüber hinaus ist ACD-A (Na-Konzentration aus Na₃Citrat: von 224,4 mmol/l) im Vergleich zum Blutplasma (Na-Konzentration: 142 mmol/l) eine stark hypernatriämische aber Kalium-freie Lösung (K-Konzentration des Blutplasmas: 4,5 mmol/l).

Obwohl versucht wird, vor dem Rückführen des Blutes in den Körper des Patienten das über die ACD-A-Lösung in das Patientenblut eingebrachte Natriumcitrat wieder zu entfernen, ist davon auszugehen, dass eine nicht unerhebliche Menge des eingesetzten Natriumcitrats im Patientenblut verbleibt und so zu einer Beeinträchtigung des physiologisch normalen Elektrolyt- und Säure-Base-Status des Patienten führen kann.

EP 0 313 808 beschreibt ein steriles, plasmafreies Lagermedium für Blutbestandteile, einschließlich roter Blutkörperchen und Blutplättchen, die entweder getrennt oder zusammen gelagert werden. Das in EP 0 313 808 beschriebene Erythrozytenlagermedium ist eine physiologisch verträgliche, wässrige Elektrolyt-Lösung, und in einem Liter dieser Elektrolyt-Lösung gibt es zwischen etwa 3,0 Gramm und etwa 25,0 Gramm Dextrose, zwischen etwa 3,0 g und etwa 6,0 g Natriumcitrat und zwischen etwa 2,0 Gramm und etwa 4,2 Gramm Natriumbikarbonat. Das in EP 0 313 808 beschriebene Erythrozytenlagermedium ist isotonisch und weist einen pH-Wert in einem Bereich von zwischen etwa 6,8 und etwa 7,4 auf.

Es ist daher auch eine Aufgabe der vorliegenden Erfindung Salzverbindungen bereitzustellen, mit denen die Blutgerinnung beispielsweise bei der therapeutischen Behandlung eines Patienten mit Hilfe eines Verfahrens zur extrakorporalen Blutbehandlung verringert werden kann, ohne den Elektrolyt-und/oder den Säure-Basen-Haushalt des Patienten nachteilig zu beeinflussen. Darüber hinaus sollen diese Salzverbindungen auch als Mittel zur Hemmung der Blutgerinnung für Zwecke der gezielten therapeutischen Verringerung der Blutgerinnung in einem Patienten dienen können. Des weiteren sollen die Salzverbindungen bei der diagnostischen Bestimmung der Blutgerinnung oder bei einem diagnostischen Verfahren, bei dem vorübergehend ungerinnbares Blut erzeugt wird, indem Calcium mit Citrat-Ionen komplexiert (gebunden) wird (Calcium-freies Blut) und anschließend die Citrat-Wirkung (z.B. durch CaCl₂-Zugabe) mit freien Ca-Ionen umgangen wird, Verwendung finden können.

Erfindungsgemäß wird die Aufgabe der vorliegenden Erfindung durch eine Zubereitung zur Hemmung der Blutgerinnung gelöst, wobei die Zubereitung eine wässrige Lösung wenigstens einer Salzverbindung ist, wobei diese Salzverbindung aus dem Kation der basischen Aminosäure Lysin oder Arginin und dem Anion der Citronensäure besteht, wobei die Zubereitung dadurch gekennzeichnet ist, dass die basische Aminosäure unter den proteinogenen Aminosäuren L-Lysin oder L-Arginin ausgewählt ist und die wässrige Lösung im Vergleich zum menschlichen Blutplasma isoton, isonatriämisch, isokaliämisch und isohydrisch ist.

"Salze" sind Verbindungen, die aus einem Anion und einem Kation bestehen. Bei einem organischen Salz ist mindestens ein Anion oder Kation eine organische Verbindung. Im Falle der vorliegenden Erfindung ist wenigstens das Kation, nämlich die basische Aminosäure eine organische Verbindung. Bei den Ausführungsformen der Erfindung, bei denen das Anion eine metabolisierbare Carbonsäure ist, sind sowohl das Kation als auch das Anion organische Verbindungen.

"Aminosäuren" sind organische Verbindungen, bestehend aus einer Aminogruppe (-NH₂), einer Carboxylgruppe (-COOH), einem Wasserstoffatom und einer organischen Seitenkette R, die an ein C-Atom gebunden sind, welches man als α-Kohlenstoffatom bezeichnet. Aminosäuren weisen die allgemeine Formel auf:

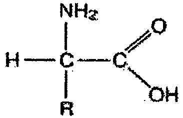

Die protonierte Aminogruppe kann als Säure und die Carboxylgruppe als Base reagieren. Bei einem bestimmten pH-Wert liegt die Aminosäure weitgehend als Zwitterion vor und verhält sich nach außen neutral. Diesen pH-Wert nennt man isoelektrischen Punkt (pH_{IP}).

Bei "basischen" Aminosäuren enthält die Seitenkette R basische funktionelle Gruppen, vorzugsweise Aminogruppen, die leicht protoniert werden können. Die Seitenketten basischer Aminosäuren können mit Säure reagieren und eine Salzbindung eingehen, das Salz besteht dann aus dem einfach positiv geladenen Aminosäure-Kation und dem Säure-Anion.

Die basischen Aminosäuren besitzen zwei basische pK_{S}-Werte und einen sauren pK_{S}-Wert. Der isolelektrische Punkt befindet sich bei basischen Aminosäuren im basischen pH-Bereich zwischen den beiden basischen pK_{S}-Werten pK_{S2} und pK_{S3} (pH_{IP} > 7).

Die basische "Seitenkette R" kann ein gerade- oder verzweigt-kettiger aliphatischer, aliphatischaromatischer, aromatischer oder heterozyklischer Rest sein, der wenigstens eine basische funktionelle Gruppe aufweist, vorzugsweise eine Aminogruppe. Vorzugsweise ist die basische Seitenkette R ein gerade- oder verzweigt-kettiger aliphatischer C₁-C₈-Alkylrest, noch bevorzugter ein gerade- oder verzweigt-kettiger aliphatischer C₁-C₄-Alkylrest.

Das "Kation" einer basischen Aminosäure ist das in der Summe nach außen hin positiv geladene Ion der jeweiligen basischen Aminosäure. Vorzugsweise handelt es sich hierbei um ein in der Summe nach außen hin einfach positiv geladenes Ion der jeweiligen basischen Aminosäure. Bei einer alternativen Ausführungsform handelt es sich um ein in der Summe nach außen hin zweifach positiv geladenes Ion der jeweiligen basischen Aminosäure.

Der "Stoffwechsef" ist die Gesamtheit der chemischen Aufbau- (Anabolismus) und Abbaureaktionen (Katabolismus), die in Lebewesen im Zusammenhang mit den Prozessen zur Aufrechterhaltung des Lebens stattfinden, z.B. Nährstoffaufnahme. Energieerzeugung, Wachstum, Bewegung. Als "metabolisierbar" werden im Zusammenhang mit der vorliegenden Erfindung solche Stoffe bezeichnet, die vom Körper des Patienten enzymatisch metabolisiert werden können. Mit anderen Worten handelt es sich bei diesen Stoffen um Substrate, die durch körpereigene Enzyme des Stoffwechselsystems des Patienten chemisch in andere Stoffe umgewandelt werden können.

Demnach handelt es sich bei den Kationen der erfindungsgemäßen Salzverbindungen um basische Aminosäuren, die durch körpereigene Enzyme des Stoffwechselsystems des Patienten chemisch in andere Stoffe umgewandelt werden können.

Bei den basischen Aminosäuren im Sinne der vorliegenden Erfindung handelt es sich um proteinogene Aminosäuren.

"Nicht-proteinogene" Aminosäuren sind im Sinne der vorliegenden Erfindung solche basischen Aminosäuren, die zwar im Stoffwechselsystem des Patienten im gesunden Zustand vorkommen würden, die jedoch nicht als Bausteine der Proteine des Patienten vorhanden sind. Beispiele für solche nicht-proteinogenen basischen Aminosäuren sind Ornithin und Citrullin (beides Stoffwechselzwischenprodukte im Hamstoffzyklus).

Vorzugsweise weisen nicht-proteinogene basische Aminosäuren keine eigene pharmakologische Wirkung auf und sind damit pharmakologisch inert.

Erfindungsgemäß handelt es sich bei den basischen Aminosäuren um proteinogene Aminosäuren, wobei als "proteinogene" Aminosäuren alle Aminosäuren bezeichnet werden, die die Bausteine der Proteine des Patienten sind, wobei es sich bei den proteinogenen Aminosäuren stets um L-Aminosäuren handelt.

Zu den derzeit bekannten proteinogenen basischen Aminosäuren zählen L-Lysin, L-Arginin und L-Histidin. Bei der vorliegenden Erfindung werden als die metabolisierbare basische Aminosäure entweder L-Lysin oder L-Arginin verwendet.

Das Anion der erfindungsgemäßen Salzverbindung ist das Anion einer metabölisierbaren Carbonsäure.

Der Begriff "metabolisierbar" wird im Zusammenhang mit der Carbonsäure der vorliegenden Erfindung in demselben Sinne verwendet, wie oben für die metabolisierbare Aminosäure definiert wurde. Demnach handelt es sich bei den Anionen der erfindungsgemäßen Salzverbindungen um Carbonsäuren, die durch körpereigene Enzyme des Stoffwechselsystems des Patienten chemisch in andere Stoffe umgewandelt werden können.

Bei der Carbonsäure im Sinne der vorliegenden Erfindung handelt es sich um eine Carbonsäure, die Zwischenprodukt oder Endprodukt im Stoffwechselsystem des Patienten im gesunden Zustand ist, nämlich Citronensäure. Weitere Beispiele für Carbonsäuren, die Zwischenprodukte oder Endprodukte im Stoffwechselsystem des Patienten im gesunden Zustand sind, sind Milchsäure, Gluconsäure und Weinsäure, weitere Carbonsäuren, die am Citratzyklus beteiligt sind, wie zum Beispiel Essigsäure, Isocitronensäure, α-Ketoglutarsäure, Bernsteinsäure, Fumarsäure, Äpfelsäure und Oxalsäure.

Die Kohlensäure (H₂CO₃) ist eine zweiprotonige Säure, deren Salze Carbonate (Anion: CO₃²⁻) bzw. Hydrogencarbonate (Anion: HCO₃⁻) heißen. Dementsprechend kann es sich bei dem Anion um das Carbonatanion oder das Hydrogencarbonatanion handeln.

Unter dem "Anion" einer metabolisierbaren Carbonsäure oder einem "Anion" der Kohlensäure ist das in der Summe nach außen hin negativ geladene Ion der jeweiligen deprotonierten Carbonsäure oder Kohlensäure zu verstehen. Vorzugsweise handelt es sich hierbei um ein in der Summe nach außen hin einfach negativ geladenes Ion der deprotonierten Carbonsäure. Bei einer alternativen Ausführungsform handelt es sich um ein in der Summe nach außen hin zweifach negativ geladenes Ion der deprotonierten Carbonsäure.

Von der vorliegenden Erfindung werden sämtliche unter den Patentanspruch 1 fallenden Kombinationen der genannten Kationen einer metabolisierbaren basischen Aminosäure mit den Anionen einer metabolisierbaren Carbonsäure umfasst. Beispiele für diese Kombinationen sind Lysincitrat und Arginincitrat. Von der vorliegenden Erfindung werden beispielsweise und ohne Beschränkung hierauf insbesondere auch die folgenden Salzverbindungen umfasst: Mono-Lysincitrat, Mono-Arginincitrat, sowie Di-Lysincitrat und Di-Arginincitrat.

Im Falle von zwei basischen Aminosäuren können die beiden Aminosäuren identisch sein oder voneinander abweichen.

Im Falle von drei-protonigen Säuren können diese in entsprechender Weise in einer, zwei oder drei Positionen Salzbindungen mit der gleichen basischen Aminosäure oder mit verschiedenen basischen Aminosäuren ausbilden. Beispielsweise werden von der vorliegenden Erfindung Salzverbindungen aus 1 Mol Citronensäure und 1 Mol Arginin (Arg-Citrat), Salzverbindungen aus 1 Mol Citronensäure und 2 Mol Arginin (Arg₂-Citrat) sowie Salzverbindungen aus 1 Mol Citronensäure und 3 Mol Arginin (Arg₃-Citrat) umfasst. Von der vorliegenden Erfindung werden daher beispielsweise und ohne Beschränkung hierauf insbesondere auch die folgenden Salzverbindungen umfasst: Tri-Lysincitrat und Tri-Arginincitrat.

Wie bereits erwähnt werden von der Erfindung auch solche Salzbindungen umfasst, bei denen drei-protonige Säuren mit verschiedenen basischen Aminosäuren kombiniert werden. So werden von der Erfindung auch beispielsweise Salzverbindungen aus 1 Mol Citronensäure in Kombination mit 2 Mol Arginin und 1 Mol Lysin (Arg₂-Lys-Citrat) umfasst. Von der vorliegenden Erfindung werden daher insbesondere auch sämtliche Kombinationen von Kationen von drei verschiedenen metabolisierbaren basischen Aminosäuren mit den Anionen der drei-protonigen metabolisierbaren Carbonsäure Citronensäure umfasst, auf deren Aufzählung hier nur deshalb verzichtet wird, da diese Kombinationen für den Fachmann ohnehin durch die hier gemachten Angaben bereits unmittelbar klar und eindeutig offenbart sind.

Die Salzverbindungen der vorliegenden Erfindung haben den Vorteil, dass sie vollständig metabolisierbar sind, d.h. dass selbst bei deren Verabreichung in großer Menge in den Blutkreislauf oder bei deren Verwendung in großer Menge im Zusammenhang mit Blutprodukten nicht die vorgenannten Nachteile der herkömmlichen für diese Zwecke verwendeten Salzverbindungen auftreten. Da die Bestandteile der erfindungsgemäßen Salzverbindungen vollständig metabolisierbar sind, können sie ohne weiteres in das Stoffwechselsystem des Patienten integriert und über diesen metabolisiert werden, ohne dass eine dauerhafte Beeinflussung des Elektrolythaushaltes und/oder des Säure-Basen-Haushaltes des Patienten zu befürchten ist.

Dementsprechend können die erfindungsgemäßen Salzverbindungen mit Vorteil zur Verwendung bei der Diagnostik oder Therapie eines Patienten angewendet werden.

Ein Beispiel für die therapeutische Verwendung der erfindungsgemäßen Salzverbindungen ist die Infusionstherapie, durch intravenöse Verabreichung größerer Flüssigkeitsmengen für Zwecke der Steigerung des intravasalen Flüssigkeitsvolumens und/oder des extrazellulären Flüssigkeitsvolumens bei der Volumenersatz-, Flüssigkeitszufuhr- und/oder Elektrolyt- bzw. Osmotherapie.

Die erfindungsgemäße Salzverbindung kann als Mittel zur Einstellung des Basenüberschusses (BE) oder des potentiellen Basenüberschusses (BEpot) in einem Patienten verwendet werden (entweder ansäuemd oder alkalisierend).

Mit dem "BE" (base excess bzw. Basenüberschuss [mmol/l]) wird beschrieben, welche Wirkung ein Stoff oder ein Stoffgemisch hat, wenn der Stoff oder das Stoffgemisch einer Blutprobe *in vitro* oder *in vivo* zugeführt wird. Ein negativer BE-Wert bedeutet, dass der zugegebene Stoff oder das zugegebene Stoffgemisch eine Säuerung des Blutes verursacht. Ein positiver BE-Wert bedeutet, dass der zugegebene Stoff oder das zugegebene Stoffgemisch zu einer Alkalisierung des Blutes führt. Der potentielle Basenüberschusses (BEpot) beschreibt zusätzlich die Wirkung beim Patienten *in vivo,* nachdem der zugegebene Stoff oder das zugegebene Stoffgemisch beim Patienten potentiell metabolisiert wurde.

Die Ergebnisse, die in Tabelle III des Beispiels Nr. 3 dargestellt sind, zeigen, dass Na₃Citrat einen BE von 0 mmol/l ermöglicht. Na₃Citrat übt originär also keinen Einfluß auf den pH-Wert bzw. BE des Blutes aus. Allerdings hat Na₃Citrat relativ großen Einfluss auf den BEpot des Patienten und die Natrium-Belastung ist erheblich. Bei der reinen Citronensäure gibt es keine Natrium-Belastung des Patienten. Die Citronensäure führt allerdings zu einem negativen, wenn auch reversiblen BE des Patienten, sobald diese metabolisiert worden ist.

Der Vorteil der erfindungsgemäßen Salzverbindung liegt darin, dass durch deren Gegenwart der BE des Patienten nicht beeinflusst wird. Vorzugsweise beträgt die Phasenabweichung (BE) des Patienten nach der Gabe des Stoffes 0 ± 10 mmol/l. Außerdem macht die potenzielle Basenabweichung (BEpot) nach der Metabolisierung des Stoffes vorzugsweise lediglich 0 ± 10 mmol/l aus.

Die erfindungsgemäße Salzverbindung kann auch zur Einstellung des pH-Werts in einer biologischen oder medizinischen Probe verwendet. Auch hier liegt der Vorteil darin, dass hierdurch der BE der Probe nicht beeinflusst wird. Beispielsweise haben 100 mmol/l ArgAcetat (pK 4,6) eine Pufferkapazität β von nur 7,0 mmol/l/pH. ArgAcetat kann aber nach beiden Seiten puffern, weil β nach beiden Seiten zunimmt. Soll also ein pH-Wert von 7,4 eingestellt werden, müssen nur 7,3 mmol/l/NaOH zugesetzt werden

Die Salzverbindurtg kann auch als Puffersubstanz verwendet werden. Hierbei ist zu beachten, dass die Pufferkapazität β der Salzverbindung umso größer ist, je höher der oberste (alkalischste) pK der Säure liegt und je mehr Arginin bzw. Lysin vorliegt (Mono-, Di-, Tri-Aminosäure-Salze). Bevorzugte Salzverbindungen sind in diesem Zusammenhang daher solche, deren oberster (alkalischster) pK-Wert > 0 ist, noch bevorzugter > 12 ist. Außerdem sind solche Salzverbindungen bevorzugt, bei denen mehr als 1 Mol basische Aminosäure pro 1 Mol Carbonsäure kombiniert sind (z.B. Arg₂Succinat). Noch bevorzugter sind solche Salzverbindungen, bei denen mehr als 2 Mol basische Aminosäure pro 1 Mol Carbonsäure kombiniert sind (z.B. Arg₃Citrat). Insbesondere bevorzugt sind solche Salzverbindungen, bei denen 2 Mol (z.B. Arg₂Succinat) oder 3 Mol (z.B. Arg₃Citrat) basische Aminosäure pro 1 Mol Carbonsäure kombiniert sind.

Die Salzverbindung kann auch zur Einstellung der Osmolalität in Infusionslösungen verwendet werden. Dies hat den Vorteil, dass hierfür der Infusionslösung keine anorganischen Elektrolyte zugeführt werden müssen, die den Elektrolythaushalt des Patienten negativ beeinflussen könnten. Die erfindungsgemäßen Salzverbindungen wirken hier sozusagen als "osmolale Platzhalter", d.h. in einer Infusionslösung kann die Osmolalität ohne Einfluß auf die Elektrolytzusammensetzung beliebig eingestellt werden.

Die erfindungsgemäßen Salzverbindungen können auch zum Zuführen von Bicarbonat-Anionen im Patienten *in vivo* verwendet werden. Der Vorteil besteht darin, dass auch hier das Zuführen von möglicherweise nachteiligen anorganischen Elektrolyten verzichtet werden kann. Außerdem besteht so die Möglichkeit, die Nachteile von anorganischem Bicarbonat (CO₂ als flüchtige Base) zu vermeiden.

Die erfindungsgemäßen Salzverbindungen können auch zur Bereitstellung von Bicarbonat in einer biologischen oder medizinischen Probe verwendet werden.

Die erfindungsgemäßen Salzverbindungen können auch für die künstliche (parenterale) Ernährung verwendet, da Sie als Mischung aus Aminosäure und u.U. organischer Carbonsäure pHneutrale Energieträger darstellen (z.B. liefert 10 g ArgAcetat 35,4 kcal; Arginin = 4 kcal/g und Acetat = 3,5 kcal/g).

Erfindungsgemäß dient die Salzverbindung zur Verwendung als Mittel zur Hemmung der Blutgerinnung. Beispielsweise kann die Salzverbindung bei der therapeutischen Verringerung der Blutgerinnung bei einem Patienten verwendet werden. Hierfür können die erfindungsgemäßen Salzverbindungen in Form einer entsprechenden Arzneimittelzubereitung dem Patienten in angemessener Dosierung intravenös verabreicht werden.

Bei einer alternativen Ausführungsform dient die erfindungsgemäße Salzverbindung zur Hemmung der Blutgerinnung bei der therapeutischen Behandlung eines Patienten mit Hilfe eines Verfahrens zur extrakorporalen Blutbehandlung, indem dem zu behandelnden Blut des Patienten die erfindungsgemäße Salzverbindung zugegeben wird. Beispiele für Verfahren zur extrakorporalen Blut-Behandlung sind Behandlungen mit einer Herz-Lungen-Maschine (HLM), die extrakorporale Membranoxygenierung (ECMO), die kontinuierliche Nierenersatztherapie (CRRT), wie z.B. Hämofiltration (CVVH), Hämodialyse (CWHD), Hämodiafiltration (CVVHDF), und die intermittierende Nierenersatztherapie oder intermittierende Hämodialyse (IHD). Bei diesen Verfahren können die erfindungsgemäßen Salzverbindungen in Form einer entsprechenden Zubereitung dem Patientenblut extrakorporal in angemessener Dosierung zugesetzt werden, wobei der Vorteil der erfindungsgemäßen Salzverbindungen insbesondere darin besteht, dass die Salzverbindungen vor dem Zurückführen des extrakorporal behandelten Blutes aus dem Blut nicht wieder vollständig entfernt werden müssen, da die Verbindungen keinen unerwünschten Einfluss auf den Elektrolytund Säure-Basen-Haushalt des Patienten haben und vollständig von dessen Stoffwechselsystem metabolisierbar sind: Nicht entfernte, beim Patienten verbleibende Anteile ändern - im Gegensatz zum herkömmlichen Citrat - unabhängig von der Verdünnung im Patientenblut nicht den BE, den BEpot, die Elektrolytkonzentrationen oder die Osmolalität des Patienten.

Die vorliegende Erfindung umfasst auch die Verwendung der erfindungsgemäßen Salzverbindung als diagnostisches Mittel zur Hemmung der Blutgerinnung bei einem *in* vitro-Verfahren der Blutgerinnungsdiagnostik oder bei einem anderen diagnostischen *in vitro*-Verfahren, vor allem dann, wenn bei dem Verfahren vorübergehend ungerinnbares Blut erzeugt wird, indem Calcium mit Citrat-Ionen komplexiert (gebunden) wird (Calcium-freies Blut) und anschließend die Citrat-Wirkung mit freien Ca-Ionen umgangen wird (z.B. durch CaCl₂-Zugabe). Im Zusammenhang mit diesen Ausführungsformen werden die erfindungsgemäßen Salzverbindungen in Form einer entsprechenden diagnostischen Zubereitung der Probe in geeigneter Dosierung zugesetzt.

Im Verlaufe einer Blutgerinnungs-Diagnostik sollte, beginnend mit der Blutentnahme (Präanalytik) keine Veränderungen am pH-Wert der zu untersuchenden Probe vorgenommen werden, da jede pH-Änderung das diagnostische Ergebnis beeinflussen muss (Zander: DE 10 2008 022 884, WO 2009/135785 und EP 09 742 003). Dazu eignet sich prinzipiell eine reine Citratlösung, die aufgrund ihrer 3 pK-Werte beim Blut-pH-Wert von ca. 7,40 praktisch keine Pufferkapazität besitzt. Eine solche Lösung weist jedoch einen pH-Wert von ca. 8,5 auf. Werden hingegen Lösungen mit einem Zusatz von Citronensäure eingesetzt, als gepufferte Lösungen bezeichnet, werden die pH-Werte der Blutprobe deutlich verändert.

Somit sollte die Forderung für eine Citratlösung lauten, dass der BE der Lösung 0 mmol/l betragen muss, damit beim Einsatz dieser Lösung keine pH-Veränderung im Blut-Lösungs-Gemisch auftritt. Die Forderung nach einem BE von 0 mmol/l, kann zusätzlich mit Bicarbonat (24 mmol/l) erfolgen, allerdings nur dann, wenn gleichzeitig keine Citronensäure vorliegt, weil Bicarbonat nur beim pH-Wert von 7-9 stabil bleibt. Ein Ausführungsbeispiel wird unten unter Beispiel 5 a) beschrieben.

Bei einer weiteren Ausführungsform der vorliegenden Erfindung werden die erfindungsgemäßen Salzverbindungen zur Hemmung der Blutgerinnung bei der Herstellung, Lagerung und Aufbereitung (z.B. Plasmapherese, Cytapherese) von Blutprodukten verwendet. Bei diesen Ausführungsformen werden die erfindungsgemäßen Salzverbindungen dem jeweiligen Blutprodukt bei dessen Herstellung, Lagerung und Aufbereitung in zur Hemmung der Blutgerinnung geeigneter Dosierung zugesetzt.

Bei den vorgenannten Ausführungsformen, bei denen die erfindungsgemäße Satzverbindung als Mittel zur Hemmung der Blutgerinnung verwendet wird, handelt es sich bei dem Anion der erfindungsgemäßen Salzverbindung jeweils um das Citratanion.

Erfindungsgemäß wird die erfindungsgemäße Salzverbindung in Form einer Zubereitung verwendet, die im Vergleich zum menschlichen Blutplasma isoton, isonatriämisch, isokaliämisch und/oder isohydrisch ist. Ein Beispiel für eine solche Ausführungsform ist ein Citrat-haltiges Mittel zur Gerinnungshemmung, wobei die Zusammensetzung dem menschlichen Plasma mit Arginin-Citrat angeglichen wird: isoton (Osmolalität 288 ± 10 mosmollkg H₂O), isonatriämisch (Natrium 142 ± 10 mmol/l), isokaliämisch (Kalium 4,5 ± 2 mmol/l), isohydrisch in vitro (Base Excess BE 0 ± 10 mmol/l) und *in vivo* (potentieller Base Excess 0 ± 10 mmol/l). Ausführungsbeispiele werden unten unter Beispiel 5 a) und b) beschrieben. Wahlweise kann eine Lösung auch mit alkalisierender Wirkung *in vivo* (potentieller Base Excess + 5 bis + 50 mmol/l) gewählt werden.

Die Salzverbindungen der vorliegenden Erfindung können im Zusammenhang mit der Diagnose und Therapie von allen Lebewesen, die ein mit dem Menschen vergleichbares Blutkreislaufsystem aufweisen, verwendet werden. Vorzugsweise handelt es sich bei dem Patienten um ein Säugetier. Noch bevorzugter handelt es sich bei dem Patienten um einen Menschen.

Die vorliegende Erfindung umfasst auch Zubereitungen, die wenigstens eine erfindungsgemäße Salzverbindung umfassen und zusätzlich wenigstens einen pharmazeutisch verträglichen Träger-Hilfs- oder Verdünnungsstoff enthalten. Die im Zusammenhang mit den oben genannten Anwendungen in Frage kommenden Träger-, Hilfs- oder Verdünnungsstoffe sind dem Fachmann wohlbekannt und deren jeweilige Eignung für die bestimmten Zwecke der vorliegenden Erfindung und die dabel zum Einsatz kommenden Mengen hängen von der spezifischen Aufgabenstellung des speziellen im Rahmen der vorliegenden Erfindung zu behandelnden oder zu diagnostizierenden Zustandes und der Gesamtkonstitution des Patienten ab.

Erfindungsgemäß handelt es sich bei den Zubereitungen, die wenigstens eine erfindungsgemäße Salzverbindung umfassen, um wässrige Lösungen wenigstens einer erfindungsgemäßen Salzverbindung. Besonders bevorzugt sind solche wässrige Lösungen der erfindungsgemäßen Salzverbindung(en), die für die intravenöse Verabreichung an einen Patienten geeignet sind.

Bei einer Ausführungsform handelt es sich bei der Zubereitung um eine Arzneimittelzubereitung zur Verringerung der Blutgerinnung in einem Patienten. Bei einer anderen Ausführungsform handelt es sich um eine Zubereitung oder ein Medizinprodukt zur Hemmung der Blutgerinnung bei der therapeutischen Behandlung eines Patienten mit Hilfe eines Verfahrens zur extrakorporalen Blutbehandlung. Bei noch einer anderen Ausführungsform handelt es sich bei der Zubereitung um ein Additiv zu einem Blutprodukt. Bei einer weiteren Ausführungsform handelt es sich um eine Zubereitung oder diagnostische Zusammensetzung zur Hemmung der Blutgerinnung bei der Blutgerinnungsdiagnostik oder bei einem anderen diagnostischen Verfahren, bei dem ungerinnbares Blut untersucht wird. Bei weiteren Ausführungsformen handelt es sich bei der Zubereitung um eine Infusionslösung.

Unter den Begriff "Arzneimittel" fallen im Zusammenhang mit der vorliegenden Erfindung solche Stoffe oder Zubereitungen aus Stoffen, die zur Anwendung im oder am menschlichen oder tierischen Körper bestimmt sind und als Mittel mit Eigenschaften zur Heilung oder Linderung oder zur Verhütung menschlicher oder tierischer Krankheiten oder krankhafter Beschwerden bestimmt sind oder die im oder am menschlichen oder tierischen Körper angewendet oder einem Menschen oder einem Tier verabreicht werden können, um entweder die physiologischen Funktionen durch eine pharmakologische, immunologische oder metabolische Wirkung wiederherzustellen, zu korrigieren oder zu beeinflussen oder eine medizinische Diagnose zu erstellen.

Unter den Begriff "Arzneimittel" fallen im Zusammenhang mit der vorliegenden Erfindung allerdings keine Stoffe oder Zubereitungen aus Stoffen, die unter die Definition des Begriffs "Medizinprodukt" fallen.

Der Begriff "Medizinprodukt" umfasst im Zusammenhang mit der vorliegenden Erfindung alle Stoffe oder Stoffgemische, die zur Anwendung am Patienten für die Erkennung, Verhütung, Überwachung, Behandlung oder Linderung von Krankheiten, die Erkennung, Überwachung, Behandlung, Linderung oder Kompensierung von Verletzungen oder Behinderungen oder die Untersuchung, den Ersatz oder die Veränderung eines physiologischen Vorgangs bestimmt sind, und deren bestimmungsgemäße Hauptwirkung im oder am menschlichen Körper weder durch pharmakologische oder immunologische Mittel noch metabolisch erreicht wird, deren Wirkungsweise aber durch solche Mittel unterstützt oder erst möglich werden kann.

"Diagnostika", "Diagnosemittel" oder "diagnostische Zusammensetzungen" sind im Rahmen der vorliegenden Erfindung Stoffe oder Stoffgemische, die zur Untersuchung des Zustandes und der Funktion des Organismus eines Patienten verwendet werden. Sie dienen auch der Krankheitsverlaufskontrolle, der Therapiekontrolle und der Therapiesteuerung. Diese Stoffe oder Stoffgemische werden entweder außerhalb des Organismus auf aus dem Körper des Patienten entnommene bzw. vom Körper abgegebene Proben angewendet ("*in vitro*-Diagnostika") oder müssen dem Patienten für Diagnosezwecke verabreicht werden ("*in vivo*-Diagnostika").

"Blutprodukte" sind im Zusammenhang mit der vorliegenden Erfindung aus Patientenblut gewonnene Blutbestandteile oder Vollblut, die zur Übertragung (Transfusion) an einen Empfänger aufbereitet worden sind, wie z.B. Erythrozyten-Konzentrat, gefrorenes Frischplasma (GFP), Thrombozyten-Konzentrat, Granulozyten-Konzentrat, thrombozytenreiches Plasma, Stammzellpräparate und Albumin.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Begebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Des weiteren wird darauf hingewiesen, dass es für den Fachmann selbstverständlich ist, dass die in der vorliegenden Anmeldung angegebenen Ausführungsbeispiele, wie beispielsweise in den nachfolgenden Beispielen angegeben, lediglich dazu dienen, die als Ausführungsbeispiele wiedergegebenen möglichen Ausführungsformen der vorliegenden Erfindung exemplarisch anzugeben. Der Fachmann wird daher ohne weiteres verstehen, dass darüber hinaus auch alle anderen Ausführungsformen, die die in den Ansprüchen genannten erfindungsgemäßen Merkmale oder Merkmalskombinationen aufweisen, innerhalb des Schutzumfangs der Erfindung liegen. Auf die umfassende, explizite Darstellung sämtlicher denkbarer Ausführungsformen wird auch in diesem Zusammenhang hier nur der Kürze und der Lesbarkeit der Beschreibung wegen verzichtet.

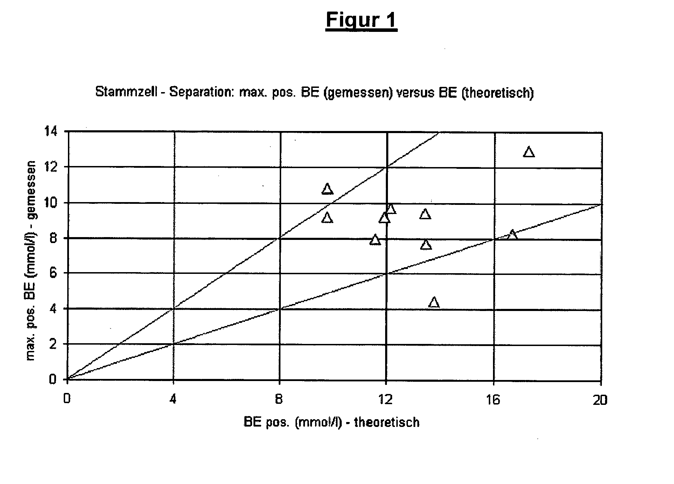

Die dieser Anmeldung anhängende
- Figur 1 zeigt:: eine graphische Darstellung der Ergebnisse eines Versuchs zur Untersuchung des Einflusses der derzeit üblichen ACD-A Lösung auf den Säure-Basen-Haushalt von Patienten unter Cytapherese.

### Beispiele

### 1. Puffereigenschaften der Salzverbindungen

In der folgenden Tabelle I sind Beispiele von erfindungsgemäßen (*) und nichterfindungsgemäßen Salzverbindungen unter Angabe von deren speziellen Puffereigenschaften aufgeführt.

**Tabelle I: Metabolisierbare Anionen von basischen Aminosäuren; Säure-Basen-Daten (37 °C)**

| Ansatz | pK-Werte | Zusatz | Gem. | Berechnete Daten | | |
|---|---|---|---|---|---|---|
| | | Säure mol/l | pH | NaOH ΔpH 7,2-7,6 mmol/l | ß (bei 7,4) mmol/l/pH | Titr. auf 7,4 mmol/l |
| Arginin | 2,0 / 9,0 / 12,1 | 0,1 | 10,26 | | | |
| -Bicarbonat | Kohlensäure | + CO₂ | 7,0 bis | 7,5 je nach Arginin-Konzentration | | |
| | 6,1 | | | | | |
| -Acetat | Essigsäure | + 0,1 | 5,99 | 2,8 | 7,0 | 7,3 |
| | 4,6 | | | | | |
| -Lactat | Milchsäure | + 0,1 | | | | |
| | 3,8 | | | | | |
| -Malat | Äpfelsäure | + 0,2 | 6,67 | 5,4 | 13,5 | 5,8 |
| | 3,5 / 4,7 | | | | | |
| -Tartrat | Weinsäure | + 0,2 | | | | |
| | 3,0 / 4,3 | | | | | |
| -Citrat (*) | Citronensäure | + 0,3 | 6,99 | 7,5 | 18,8 | 6,5 |
| | 3.0 / 4.4 / 5.8 | | | | | |
| Lysin | 2,2 / 8,9 / 10,3 | 0,1 | 9,55 | | | |
| -Bicarbonat | Kohlensäure | + CO₂ | | | | |
| | 6,1 | | | | | |
| -Acetat | Essigsäure | + 0,1 | 5,99 | 2,5 | 6,3 | 6,8 |
| | 4,6 | | | | | |
| -Lactat | Milchsäure | + 0,1 | | | | |
| | 3,8 | | | | | |
| -Malat | Äpfelsäure | + 0,2 | 6,52 | 4,0 | 10,0 | 4,7 |
| | 3,5 / 4,7 | | | | | |
| -Tartrat | Weinsäure | + 0,2 | | | | |
| | 3,0 / 4,3 | | | | | |
| -Citrat (*) | Citronensäure | + 0,3 | 6,88 | 5,9 | 14,8 | 5.8 |
| | 3,0 / 4,4 / 5,8 | | | | | |

| | | | | | | |
|---|---|---|---|---|---|---|
| Erläuterungen zur Tabelle 1. NaOH mmol/l für ΔpH von 7,2-7,6: Wie viel NaOH in mmol/l ist für dieses ΔpH nötig. 2. ß (mmol/l/pH): Die daraus berechnete Pufferkapazität bei pH 7.40. 3. Titr. auf 7,4 (mmol/l): Wie viel NaOH ist nötig, einen pH-Wert von 7,4 einzustellen. 4. Arga-Citrat wurde einfach kristallisiert, nach quantitativer Zugabe von H₂O wurden die Ausgangswerte des pH und der Osmolalität wieder gefunden. | | | | | | |

Aus den vorliegenden Daten können folgende Rückschlüsse gezogen werden:
1. Die Pufferkapazität ß ist umso größer, je höher der oberste (alkalischste) pK der Säure und je mehr Arginin bzw. Lysin (Mono-, Di-, Tri-) vorliegt. Dies ermöglicht eine gezielte Auswahl.
2. Soll ein physiologischer pH-Wert von 7,4 in einer 0,1 molaren Lösung eingestellt werden, so sind in der Spalte Titration pH = 7,4 die entsprechenden Zusätze angegeben. Es wird also angegeben, wie viel NaOH (mmol/l) zuzusetzen ist, um diesen pH-Wert einzustellen.
3. Für die untersuchten 0,1 molaren Lösungen wird gezeigt, dass deren Pufferkapazität dann gering ausfällt, wenn man zum Vergleich den ß-Wert für Normalblut (pH 7,2 - 7,6) mit ca. 72 mmol/l/pH aufführt. Diese Betrachtung ist dann wichtig, wenn man im Falle von Arg₃-Citrat eine ungepufferte Gerinnungshemmung mit einer 0,1 molaren Lösung (100 mmol/l) erzeugen will, eine Lösung, die üblicherweis im Blut 1:10 verdünnt wird, was dann eine Pufferkapazität ß von nur noch 1,9 mmol/l/pH ergibt (im Vergleich zu 72 mmol/l/pH).
4. Obwohl metabolisch umsetzbar haben diese Substanzen keine metabolische Wirkung auf den Säure-Basen-Haushalt, Dies wird durch folgende Vergleiche deutlich:
   - Essigsäure (CH₃COOH) wirkt reversibel als Säure (nach Metabolisierung neutral), Natriumacetat dagegen wirkt irreversibel alkalisierend.
   - Arg • HCl (Argininhydrochlorid) wirkt als Säure (HCl ist nicht metabolisierbar), Arg • HCO₃ wirkt dagegen neutral, ebenso Tri-Arg-Citrat u. a.
   - Ammonium-Chlorid (NH₃+HC)=NH₄Cl) wirkt als Säure (HCl nicht metabolisierbar) Ammonium-Bicarbonat (NH₃+H₂CO₃=NH₄HCO₃) wirkt dagegen neutral.

### 2. Osmotischer Koeffizient

### a) Osmotische Koeffizienten von erfindunasoemäßen (*) und nicht erfindungsgemäßen Verbindingen

**Tabelle II: Osmotische Koeffizienten (0,1 molare Lösungen)**

| Substanz | Osmot. Wertigkeit | Osmot. Koeffizient | |
|---|---|---|---|
| Glucose | 1 | 1,013 | (Literatur) |
| Lysin | 1 | 1,000 | |
| Arginin | 1 | 0,980 | |
| NaCl | 2 | 0,926 | (Literatur) |
| NaHCO₃ | 2 | 0,926 | (Annahme) |
| Arg-HCl | 2 | 0,895 | |
| Lys₂-Malat | 3 | 0,847 | |
| Arg-HCO3 | 2 | 0,833 | 0,2 m 0,833 100 % CO₂ bei 37 °C |
| | | | 0,3 m 0,763 100 % CO₂ bei 37 °C |
| Arg₂-Malat | 3 | 0,783 | |
| Lys₃-Citrat (*) | 4 | 0,715 | |
| K₃Citrat | 4 | 0,681 | |
| Na₃Citrat | 4 | 0,681 | |
| Arg₃-Citrat (*) | 4 | 0,593 | |
| Citronensäure | 3 | 0,555 | |

Beispiele: Eine 0,1 molare Argininlösung zeigt eine Osmolalität von 98 mosmol/kg H₂O, eine 0,1 molare Arg₃-Citratlösung eine Osmolalität von 237,2 mosmol/kg H₂O.

### Zum Vergleich:

Typische, derzeit in der *in vitro*-Diagnostik und *in vivo*-Therapie eingesetzte Lösungen sind:
1. Citratlösung 109 mmol/l, pH = ca. 8,3 (37 °C) (schlecht definiert, da ungepuffert), hergestellt aus 3,21 % (w/v) Tri-Natrium-Citrat-Dihydrat.
Messwert: Osmolalität: 297 mosmol/kg H₂O, pH (37°C) = 8,81 gerade noch isotone Lösung.
2. Citrat-Citronensäure-Lösung 109 mmol/l Citrat,
mit 85,5 mmol/l Tri-Natriumcitrat (MW 294,1) plus 23,4 mmol/l Citronensäure,
pH = 5,6 (37°C), hergestellt aus 2,515 % (w/v) Tri-Natriumcitrat-Dihydrat plus 0,492 % (w/v) Citronensäure-Monohydrat (MW 210,1).
Messwert: Osmolalität: 272 mosmol/kg H₂O, pH (37°C) = 5,42.
stark hypotone Lösung.
3. ACD-A

| Lösung | Citrat | Citr-Sr. | Ges.-Citrat | Messwert Osmolalität |
|---|---|---|---|---|
| ACD-A | 74,8 | 38,1 | 112,9 | 265 mosmol/kg H₂O |
| Mit | 24,5 g/l Glucose | | | 396 mosmol/kg H₂O |

Kommentar zur Glucose, die in der Lösung vorhanden ist:
Obwohl bei der Messung einer Lösung erfasst, wird die Glucose - auch *in vitro -* osmotisch nicht wirksam, da sie die Osmolalität sowohl im Plasmaals auch im Ery-Wasser gleichermaßen erhöht. ACD-A ist eine stark hypotone Lösung.

### 3. Einfluss von Citronensäure und Natriumcitraten auf BE, BEpot und die Natriumkonzentration

Eine typische Verdünnung von 1:10 (9 Teile Blut + 1 Teil Citratlösung) ergibt für die verschiedenen Citratformen folgende Wirkungen, wenn eine typische Lösung mit 100 mmol/l für die Gerinnungshemmung eingesetzt würde:

**Tabelle III: Auswirkungen verschiedener herkömmlicher Citratformen auf den BEpot**

| | | BE (mmol/l) | BEpot (mmol/l) | Na-Zufuhr (mmol/l) |
|---|---|---|---|---|
| 10 mmol/l | Citronensäure | -30 | 0 | 0 |
| 10 mmol/l | NaH₂Citrat | -20 | + 10 | 10 |
| 10 mmol/l | Na₂HCitrat | -10 | + 20 | 20 |
| 10 mmol/l | Na₃Citrat | 0 | + 30 | 30 |

Die Ergebnisse aus Tabelle III zeigen, dass Na₃Citrat einen BE von 0 mmol/l ermöglicht. Na₃Citrat übt originär also keinen Einfluß auf den pH-Wert bzw. BE des Blutes aus. Allerdings hat Na₃Citrat einen sehr großen Einfluss auf den BEpot des Patienten und die Natrium-Belastung ist erheblich, wenngleich dies zumindest *in vitro* keine Rolle spielt, Isotonie unterstellt. Bei der reinen Citronensäure entfällt die Natrium-Belastung des Patienten. Die Citronensäure führt allerdings zu einem negativen, wenn auch reversiblen BE des Patienten, sobald die Citronensäure metabolisiert worden ist.

### 4. Einfluss der nicht erfindungsgemäßen Salzverbindungen auf den Säure-Basen-Haushalt

### Beispiel ACD-A

| Lösung | Citrat | Citr-Sr. | Ges.-Citrat | pH (37°C) gemessen | Ableitung (mmol/l) | |
|---|---|---|---|---|---|---|
| | | | | | BE | BEpot |
| ACD-A | 74,8 | 38,1 | 112,9 | 4,96 | | |
| | | - 114,3 (reversibel) | | | - 24 (HCO₃) | |
| | | | | ges. | -138 | |
| | 224,4 (-24) | | | ges. | | + 200 |

Eine primär nach Infusion reversibel stark säuernde Lösung (BE - 138 mmol/l, Citronensäure), sekundär nach potentieller Metabolisierung stark alkalisierend (BEpot + 200 mmol/l. Citrat).

Es wurde eine maschinelle Stammzell-Separation über ca. 3 Stunden unter Verwendung von ACD-A durchgeführt. Im Mittel haben alle 10 Spender 829 ml ACD-A erhalten, die im Extrazellularraum von 14,8 l (20 % des mittleren Körpergewichts von 74,1 kg) einen reversiblen, Citronensäure-bedingten negativen BE von - 7,7 mmol/l (138,3 mmol/l x 0,829 l / 14,8 l) und einen potentiellen, Citrat-bedingten positiven BE von + 11,3 mmol/l (200,4 mmol/l x 0,829 l / 14,8 l) erzeugen müssten. Beide Wirkungen können sich aber wegen der Zeitcharakteristik des Metabolismus von Citronensäure (reversible Ansäuerung) und Citrat (irreversible Alkalisierung) überschneiden. Da die Alkalisierung aber überwiegt, ist mit positiven BE-Werten zu rechnen. In Figur 1 sind die bei den Patienten gemessenen maximalen positiven BE-Werte im Vergleich zum vorhergesagten theoretischen BE für einen 50-100 % Citrat-Metabolismus dargestellt. Offensichtlich liegen fast alle maximalen positiven BE-Werte zwischen den Vorhersagen für einen 50 %igen und 100 %i-gen Umsatz im Patienten.

### 5. Beispielrezepturen (erfindungsgemäße (*) und nicht erfindungsgemäße)

### a) Einsatz in vitro: Gesamt-Citrat 97,2 mmol/l (für eine spätere Verdünnung von 1:10)

| | | |
|---|---|---|
| NaHCO₃ | 24,0 | mmol/l |
| K₃Citrat | 1,5 | mmol/l |
| Na₃Citrat (*) | 39 | mol/l |
| Arg₃-Citrat (*) | 56,7 | mmol/l |

Messwerte: pH (37°C) 7,25. Osmolalität 293 mosmol//kg H₂O
Berechnete Werte: Pufferkapazität (pH 7-8) ~ 0 mmol/l/pH, BE 0 mmol/l
Eigenschaften: Iso-tonisch (288), iso-kaliämisch (4,5), iso-natriämisch (141), einfach Isohydrisch (BE 0 mmol/l)

### b) Einsatz in vivo: Gesamt-Citrat 100 mmol/l

| | | |
|---|---|---|
| NaHCO₃ | 24,0 | mmol/l |
| KCl | 4,5 | mmol/l |
| Arg₃-Citrat (*) | 100 | mmol/l |

Messwerte: pH (37°C) 7,12, Osmolalität 284 mosmol/kg H₂O
Berechnete Werte: Pufferkapazität (pH 7-8) ~ 0 mmol / l / pH-, BE 0 mmol/l, BEpot 0 mmol/l Eigenschaften; Iso-tonisch (288), iso-kaliämisch (4,5), zweifach isohydrisch (BE 0, BEpot 0)

### 5. Beispiel für die Herstellung von Arg₃-Citrat

Zuerst wird Citronensäure in Wasser gelöst, dann wird Arginin (3-fach molar) zugeben. Nachdem die Pufferung Citronensäure vs. Arginin abgewartet wurde, erfolgt eine Prüfung des pH-Werts, dann Zusatz von Citrat bzw. Chlorid, zuletzt NaHCO₃ (nach Pufferung), damit keine freien H⁺- Ionen vorliegen, die HCO₃ austreiben könnten, dann Zugabe H₂O.

### 6. Verwendung der Satzverbindungen zum Zuführen von Bicarbonat

Wird eine Lösung mit 48 mmol/l Arginin mit 24 mmol/l Essigsäure versetzt, entsteht eine Mischung aus je 24 mmol/l Arginin und Arginin-Acetat mit einem pH von ca. 8,6. Dieser pH entspricht dem effektiven pK von Arginin (lonenstärke 160 mmol/l und 37 °C). Wird diese Lösung nach Infusion im Patienten zwangsläufig auf einen pCO₂ von ca. 40 mmHg äquilibriert, werden maximal 24 mmol/l Arginin-HCO₃ entstehen. Somit werden sich die physiologischen Werte von ca. 24 mmol/l HCO₃ und pH ca. 7,4 einstellen (zum Vergleich Arginin-Äquilibrierung mit reinem CO₂ in Tab. 1).

## Patentansprüche

1. Zubereitung zur Hemmung der Blutgerinnung, wobei die Zubereitung eine wässrige Lösung wenigstens einer Salzverbindung aus
a) dem Kation der basischen Aminosäure Lysin oder Arginin und
b) dem Anion der Citronensäure
ist, **dadurch gekennzeichnet, dass** die basische Aminosäure unter den proteinogenen Aminosäuren L-Lysin oder L-Arginin ausgewählt ist und die wässrige Lösung im Vergleich zum menschlichen Blutplasma isoton, isonatriämisch, isokaliämisch und isohydrisch ist.

2. Zubereitung nach Anspruch 1, **dadurch gekennzeichnet, dass** die wenigstens eine Salzverbindung aus Mono-Lysincitrat, Mono-Arginincitrat, Di-Lysincitrat, Di-Arginincitrat, Tri-Lysincitrat, Tri-Arginincitrat, ausgewählt ist.

3. Zubereitung nach Anspruch 1, **dadurch gekennzeichnet, dass** wenigstens eine Salzverbindung Di-Arginin-Mono-Lysincitrat (Arg₂-Lys-Citrat) ist.

4. Zubereitung nach einem der Ansprüche 1 bis 3, **dadurch gekennzeichnet, dass** die Basenabweichung (BE) des Patienten nach der Verabreichung der Zubereitung 0 +/- 10 mmol/l beträgt.

5. Zubereitung nach einem der Ansprüche 1 bis 4, **dadurch gekennzeichnet, dass** die potenzielle Basenabweichung (BEpot) nach der Metabolisierung der Zubereitung 0 +/- 10 mmol/l beträgt.

6. Zubereitung nach einem der Ansprüche 1 bis 5 **dadurch gekennzeichnet, dass** die Zubereitung als Mittel zur Hemmung der Blutgerinnung bei der therapeutischen Verringerung der Blutgerinnung in einem Patienten, bei der therapeutischen Behandlung eines Patienten mit Hilfe eines Verfahrens zur extrakorporalen Blutbehandlung, bei der diagnostischen Bestimmung der Blutgerinnung *in vitro* oder bei einem anderen diagnostischen *in vitro-*Verfahren verwendet wird, bei dem vorübergehend ungerinnbares Blut erzeugt wird, indem Calcium mit Citrat-Ionen komplexiert wird, wobei das Anion der Salzverbindung jeweils Citrat ist.

7. Zubereitung nach einem der Ansprüche 1 bis 6, **dadurch gekennzeichnet, dass** die Verwendung der Zubereitung zur Hemmung der Blutgerinnung bei der therapeutischen Behandlung eines Patienten mit Hilfe eines Verfahrens zur extrakorporalen Blutbehandlung erfolgt, wobei das Verfahren zur extrakorporalen Blut-Behandlung unter den folgenden ausgewählt ist: Behandlung mit einer Herz-Lungen-Maschine (HLM), extrakorporale Membranoxygenierung (ECMO), kontinuierliche Nierenersatztherapie (CRRT), Hämofiltration (CWH), Hämodialyse (CVVHD), Hämodiafiltration (CWHDF) und intermittierende Nierenersatztherapie oder intermittierende Hämodialyse (IHD).

8. Zubereitung nach einem der Ansprüche 1 bis 5 **dadurch gekennzeichnet, dass** die Zubereitung zur Hemmung der Blutgerinnung bei der Herstellung, Lagerung und Aufbereitung von Blutprodukten verwendet wird, wobei das Anion der Salzverbindung jeweils Citrat ist.

9. Zubereitung nach einem der Ansprüche 1 bis 7, **dadurch gekennzeichnet, dass** der Patient ein Mensch oder ein Säugetier ist.

10. Zubereitung nach einem der Ansprüche 1 bis 9, **dadurch gekennzeichnet, dass** die Zubereitung eine Arzneimittelzubereitung, ein Medizinprodukt, eine diagnostische Zusammensetzung für die *in vitro-* bzw. *in vivo*-Diagnostik oder ein Blutprodukt ist.

11. Zubereitung nach einem der Ansprüche 1 bis 10, **dadurch gekennzeichnet, dass** die Zubereitung eine wässrige Lösung ist, die für die intravenöse Verabreichung an einen Patienten geeignet ist.

12. Zubereitung nach einem der Ansprüche 1 bis 11, **dadurch gekennzeichnet, dass** die Zubereitung zusätzlich wenigstens einen pharmazeutisch verträglichen Träger-, Hilfs- oder Verdünnungsstoff enthält.

13. Zubereitung nach einem der Ansprüche 1 bis 12, **dadurch gekennzeichnet, dass** das die wenigstens eine Salzverbindung L-Arginin-Citrat ist und die Zubereitung dem menschlichen Plasma mit L-Arginin-Citrat wie folgt angeglichen wird: isoton mit einer Osmolalität von 288 ± 10 mosmol/kg H₂O, isonatriämisch mit 142 ± 10 mmol/l Natrium, isokaliämisch mit 4,5 ± 2 mmol/l Kalium, isohydrisch *in vitro* mit einem Base Excess BE von 0 ± 10 mmol/l und *in vivo* mit einem potentiellen Base Excess BEpot von 0 ± 10 mmol/l.

## Claims

1. Preparation for inhibiting blood coagulation, wherein the preparation is an aqueous solution of at least one salt compound of:
a) the cation of the basic amino acid lysine or arginine, and
b) the anion of citric acid,
**characterised in that** the basic amino acid is selected from the proteinogenic amino acids L-lysine or L-arginine, wherein the aqueous solution is isotonic, isonatriaemic, isokaliaemic and isohydric in comparison with human blood plasma.

2. Preparation according to claim 1, **characterised in that** the at least one salt compound is selected from mono-lysine citrate, mono-arginine citrate, di-lysine citrate, di-arginine citrate, tri-lysine citrate, tri-arginine citrate.

3. Preparation according to claim 1, **characterised in that** at least one salt compound is di-arginine mono-lysine citrate (Arg₂-Lys-citrate).

4. Preparation according to one of claims 1 to 3, **characterised in that** the base excess (BE) of the patient after administration of the preparation is 0 ± 10 mmol/l.

5. Preparation according to one of claims 1 to 4, **characterised in that** the potential base excess (BEpot) after metabolisation of the preparation is 0 ± 10 mmol/l.

6. Preparation according to one of claims 1 to 5, **characterised in that** the preparation is used as an agent for inhibiting blood coagulation in the therapeutic reduction in blood coagulation in a patient, in the therapeutic treatment of a patient by means of a method of extracorporeal blood treatment, in diagnostic determination of blood coagulation *in vitro* or in another diagnostic *in vitro* method, in which temporarily uncoagulatable blood is produced by calcium being complexed with citrate ions, wherein the anion of the salt compound is citrate, respectively.

7. Preparation according to one of claims 1 to 6, **characterised in that** the use of the preparation for inhibiting blood coagulation in the therapeutic treatment of a patient is carried out by means of a method of extracorporeal blood treatment, wherein the method of extracorporeal blood treatment is selected from the following: treatment with a heart-and-lung machine (HLM), extracorporeal membrane oxygenation (ECMO), continuous renal replacement therapy (CRRT), hemofiltration (CVVH), hemodialysis (CVVHD), hemodiafiltration (CVVHDF) and intermittent renal replacement therapy or intermittent hemodialysis (IHD).

8. Preparation according to one of claims 1 to 5, **characterised in that** the preparation for inhibiting blood coagulation is used in the production, storage and preparation of blood products, wherein the anion of the salt compound is citrate, respectively.

9. Preparation according to one of claims 1 to 7, **characterised in that** the patient is a human being or a mammal.

10. Preparation according to one of claims 1 to 9, **characterised in that** the preparation is a drug preparation, a medical product, a diagnostic composition for *in vitro* or *in vivo* diagnostics or a blood product.

11. Preparation according to one of claims 1 to 10, **characterised in that** the preparation is an aqueous solution which is suitable for intravenous administration to a patient.

12. Preparation according to one of claims 1 to 11, **characterised in that** the preparation additionally comprises at least one pharmaceutically acceptable carrier material, adjuvant or dilution substance.

13. Preparation according to one of claims 1 to 12, **characterised in that** the at least one salt compound is L-arginine citrate and the preparation is adjusted to the human plasma with the help of L-arginine citrate as follows: isotonic with an osmolality of 288 ± 10 mosmol/kg H₂O, isonatriaemic with 142 ± 10 mmol/l sodium, isokaliaemic with 4.5 ± 2 mmol/l potassium, isohydric *in vitro* with a base excess BE of 0 ± 10 mmol/l and *in vivo* with a potential base excess BEpot of 0 ± 10 mmol/l.

## Revendications

1. Préparation destinée à inhiber la coagulation sanguine, dans laquelle la préparation est une solution aqueuse d'au moins un composé de sel composé
a) du cation de l'acide aminé basique lysine ou arginine, et
b) de l'anion de l'acide citrique,
**caractérisée en ce que** l'acide aminé basique est sélectionné parmi les acides aminés protéinogènes L-lysine et L-arginine et **en ce que** la solution aqueuse est, par rapport au plasma sanguin humain, isotonique, isosodique, isopotassique et isohydrique.

2. Préparation selon la revendication 1, **caractérisée en ce que** le composé de sel, au moins au nombre de un, est sélectionné parmi le mono-citrate de lysine, le mono-citrate d'arginine, le di-citrate de lysine, le di-citrate d'arginine, le tri-citrate de lysine, le tri-citrate d'arginine.

3. Préparation selon la revendication 1, **caractérisée en ce qu'**au moins un composé de sel est le di-arginine-mono-citrate de lysine (Arg₂-Lys-citrate).

4. Préparation selon l'une des revendications 1 à 3, **caractérisée en ce que** l'excès de base (BE) du patient après administration de la préparation est de 0 ± 10 mmol/l.

5. Préparation selon l'une des revendications 1 à 4, **caractérisée en ce que** l'excès de base potentiel (BEpot) du patient après métabolisation de la préparation est de 0 ± 10 mmol/l.

6. Préparation selon l'une des revendications 1 à 5, **caractérisée en ce que** la préparation est utilisée en tant qu'agent permettant d'inhiber la coagulation sanguine dans le cadre de la réduction thérapeutique de la coagulation sanguine chez un patient, dans le cadre du traitement thérapeutique d'un patient à l'aide d'un procédé de traitement extracorporel du sang, dans le cadre de la détermination diagnostique de la coagulation sanguine *in vitro* ou dans le cadre d'un autre procédé diagnostique *in vitro,* dans lequel du sang incoagulable est généré de façon transitoire, dans lequel du calcium est complexé à des ions citrate, l'anion du composé de sel étant respectivement le citrate.

7. Préparation selon l'une des revendications 1 à 6, **caractérisée en ce qu'**il est fait usage de la préparation pour inhiber la coagulation sanguine dans le cadre du traitement thérapeutique d'un patient à l'aide d'un procédé de traitement extracorporel du sang, le procédé de traitement extracorporel du sang étant sélectionné parmi les suivants : traitement avec une machine coeur-poumon (HLM), oxygénation par membrane extracorporelle (ECMO), thérapie d'épuration extrarénale continue (CRRT), hémofiltration veino-veineuse continue (CVVH), hémodialyse (CVVHD), hémo-dia-filtration (CVVHDF) et thérapie d'épuration extrarénale intermittente ou hémodialyse intermittente (IHD).

8. Préparation selon l'une des revendications 1 à 5, **caractérisée en ce que** la préparation destinée à inhiber la coagulation sanguine est utilisée pour la fabrication, le stockage et la préparation de produits sanguins, l'anion du composé de sel étant respectivement le citrate.

9. Préparation selon l'une des revendications 1 à 7, **caractérisée en ce que** le patient est un homme ou un mammifère.

10. Préparation selon l'une des revendications 1 à 9, **caractérisée en ce que** la préparation est une préparation de médicament, un produit médical, une composition diagnostique destinée au diagnostic *in vitro* ou *in vivo,* ou un produit sanguin.

11. Préparation selon l'une des revendications 1 à 10, **caractérisée en ce que** la préparation est une solution aqueuse qui est appropriée pour une administration à un patient par voie intraveineuse.

12. Préparation selon l'une des revendications 1 à 11, **caractérisée en ce que** la préparation contient en outre au moins un véhicule, auxiliaire ou diluant compatible sur le plan pharmaceutique.

13. Préparation selon l'une des revendications 1 à 12, **caractérisée en ce que** le composé de sel, au moins au nombre de un, est le citrate de L-arginine et **en ce que** la préparation est adaptée comme suit au plasma humain avec du citrate de L-arginine : isotonique avec une pression osmotique de 288 ± 10 mosmol/kg de H₂O, isosodique avec 142 ± 10 mmol/l de sodium, isopotassique avec 4,5 ± 2 mmol/l de potassium, isohydrique *in vitro* avec un excès de base BE de 0 ± 10 mmol/l et *in vivo* avec un excès de base potentiel BEpot de 0 ± 10 mmol/l.
